# EUROPEAN PATENT SPECIFICATION

(11) **EP 4 049 631 B1**
(45) Date of publication and mention of the grant of the patent: **14.08.2024**
(21) Application number: 20941519.9
(22) Date of filing: 30.12.2020
(51) Int. Cl.: A61F 2/64

(54) **HYBRID KNEE PROSTHESIS HAVING MULTI-FUNCTIONAL ROTARY HYDRAULIC CYLINDER**
HYBRIDE KNIEPROTHESE MIT MULTIFUNKTIONELLEM ROTIERENDEN HYDRAULIKZYLINDER
PROTHÈSE DE GENOU HYBRIDE AYANT UN CYLINDRE HYDRAULIQUE ROTATIF MULTIFONCTIONNEL

(43) Date of publication of application: 31.08.2022
(73) Proprietor: Korea Labor Welfare Corporation Co., Ltd., Ulsan 44428 (KR)
(72) Inventor: SHIN, Hyunjun, Seo-gu Daejeon 35236 (KR); JEON, Mankee, Bucheon-si Gyeonggi-do 14622 (KR); CHOI, Jongmoon, Seodaemun-gu Seoul 03743 (KR); KIM, Jongkwon, Seo-gu Incheon 22735 (KR); PARK, Jinkuk, Suwon-si Gyeonggi-do 16413 (KR); Jung, Sung Yoon, Yeonsu-gu Incheon 22009 (KR); PARK, Sehoon, Bucheon-si Gyeonggi-do 14746 (KR); LEE, Seok Min, Bucheon-si Gyeonggi-do 14613 (KR)
(74) Representative: Maiwald GmbH
(86) International application number: PCT/KR2020/019462
(87) International publication number: WO 2022/145545

(56) References cited:
- WO-A1-2013/157965
- WO-A1-2020/203762
- CN-A- 110 368 152
- JP-A- 2011 518 633
- JP-A- 2020 199 292
- JP-B2- 6 152 523
- KR-B1- 101 621 610
- KR-B1- 101 932 343
- US-A1- 2015 223 952

## Description

### [Technical Field]

The present invention relates to a hybrid-type knee prosthesis apparatus having a multifunctional rotary hydraulic cylinder, and more specifically to a hybrid-type knee prosthesis apparatus having a multifunctional rotary hydraulic cylinder which is mounted on the femoral region of a patient with femoral amputation to enable flexion motion or extension motion by a knee joint module.

### [Background Art]

US 2015/223952 A1 describes a powered prosthetic thigh which can have a proximal portion configured to couple to a prosthetic hip socket and can have a distal portion attached to the proximal portion.

WO 2013/157965 A1 describes an active prosthetic shin intended for people after amputation of lower limb, facilitating the functioning of disabled persons in the society.

WO 2020/203762 A1 describes a joint device which purpose is to provide a joint device capable of stretching from a bent state when a load is applied.

CN 110 368 152 A describes a kind of a booster type knee joint artificial limb. For a person with a prosthetic leg such as a femoral prosthesis, it is a basic wish to take natural steps like a normal person. In particular, it is very difficult for a person with a prosthetic leg to climb stairs by alternately stepping on the left and right feet or to walk on a slope with a certain inclination. In this case, a firm knee braking function capable of preventing a fall, that is, a flexion control performance is the most important function required for wearing a prosthetic leg. In addition, if an active joint torque in an extension direction can be provided to lift the body weight upward, it can be of great help in overcoming stairs or upward slopes.

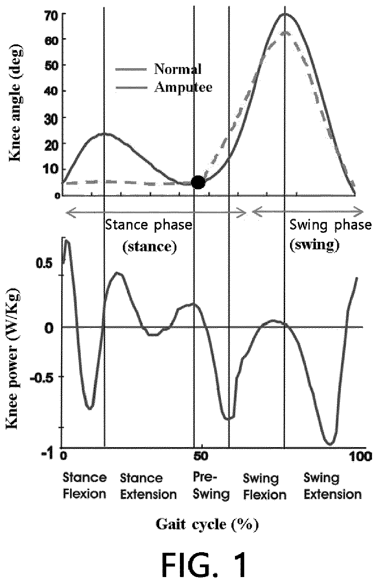

As can be seen from FIG. 1, the gait section of a person's walking is divided into a stance phase where a foot touches the ground and moves, and a swing phase section where a foot moves away from the ground. When a normal person walks, the knee goes through 2 flexions and 1 extension in the stance phase section, and 1 flexion and 1 extension in the swing phase section.

Referring to FIG. 1, in most cases of disabled persons with amputation, they pass the stance phase at a flat knee angle without stance flexion during walking, and then go directly to the swing phase section. Since the stance phase on the side of wearing a prosthesis is unstable, they have a relatively short stance phase. This is a fundamental cause of disabled persons with amputation who wear a prosthesis walking asymmetrically.

Prosthesis apparatuses for disabled persons with amputation, which have been developed to allow disabled persons with amputation to walk more naturally, are largely divided into a passive-type prosthesis, a variable damping-type prosthesis and an active-type prosthesis.

The passive-type prosthesis uses a hydraulic cylinder to provide only passive forces by the hydraulic cylinder when a disabled person with amputation is walking, and it is made only with mechanical elements, and the joint resistance values can be adjusted manually before walking, but during walking, it has only one preset joint resistance. Since such a passive-type prosthesis is manufactured only with mechanical elements, there is no problem such as battery discharge, and it has strengths in terms of reliability and durability of the prosthesis. However, since the passive-type prosthesis has only one knee resistance which is set manually during walking, its ability to adapt to walking speed is relatively low, and it consumes 60% or more energy than healthy people due to asymmetric walking patterns and hip hiking problems, and it has a disadvantage in that the walking speed is slower than that of a normal person.

The variable damping-type prosthesis is a prosthesis that is constituted with the goal of adjusting the hydraulic pressure and damping values of an MRZER damper in real time to adjust the knee joint resistance in real time to be suitable for the walking situation. Currently, the most commonly used variable damping-type prosthesis, to which a hydraulic cylinder is applied, has advantages of improving knee stability and adaptability to the difference in walking speeds, compared to the passive-type prosthesis, by controlling the hydraulic pressure inside the hydraulic cylinder through a hydraulic nozzle in real time. However, since the variable damping-type prosthesis also uses a hydraulic cylinder, which is a passive mechanical element, it cannot provide an active driving force. Therefore, there is a disadvantage in that it cannot provide all of the power necessary for daily life, because it cannot be applied in situations where force in an extension direction must be applied to the knee, such as walking or running on stairs or slopes.

The active-type prosthesis is a prosthesis using a driving motor that can provide a knee torque required for walking in an extension direction. The active-type prosthesis can provide an adequate force even when a large amount of power is required, such as walking on a slope or walking fast, and it can restore most activities of daily life. However, since the active-type prosthesis continuously uses the driving motor to move the prosthesis with power during both the stance phase and swing phase sections, it is difficult to be used continuously for a long period of time because of the large consumption of the battery. Further, there are disadvantages in that in order to generate a large force, the size of the power unit increases, the control of the system becomes complicated, and the weight is large.

Therefore, by supplementing the disadvantages of the conventional passive-type prosthesis, variable damping-type prosthesis and active-type prosthesis described above, it is necessary to develop a prosthesis apparatus that can provide adequate power when the disabled person with amputation needs more than a certain amount of power when moving not only on flat ground but also on stairs or ramps, and that can be used for a long period of time due to low energy loss. To this end, it is necessary to develop a prosthesis apparatus capable of fusing the driving methods by the conventional prosthesis apparatuses described above, actively selecting and applying one specific driving method according to the walking situation, or combining and applying multiple driving methods.

### [Disclosure]

### [Technical Problem]

An exemplary embodiment of the present invention is directed to providing a prosthesis apparatus capable of providing an appropriate torque required for movement in an extension direction when a large force is required for a prosthesis wearer during movement such as walking on flat ground as well as climbing stairs or ramps.

An exemplary embodiment of the present invention is directed to providing a prosthesis apparatus that can be used for a long period of time due to low energy loss.

An exemplary embodiment of the present invention is directed to providing a prosthesis apparatus capable of diversifying driving methods.

An exemplary embodiment of the present invention is directed to providing a prosthesis apparatus that can be easily switched between various driving methods.

### [Technical Solution]

According to an aspect of the present invention, provided is a hybrid-type knee prosthesis apparatus having a multifunctional rotary hydraulic cylinder which is mounted on the knee portion of a patient with femoral amputation to assist in walking, including a knee joint module connected to a lower leg prosthesis of the patient with femoral amputation; a rotary hydraulic cylinder operatively coupled to the knee joint module and pivotally rotating the knee joint module by pivot rotation of a rotation shaft; an electric motor for providing active power to the rotary hydraulic cylinder; a driving cable for operatively connecting the rotary hydraulic cylinder and the electric motor to each other; and a control module for controlling to operate in any one mode of a passive mode in which the rotary shaft provided in the rotary hydraulic cylinder is pivotally rotated by a hydraulic pressure formed inside the rotary hydraulic cylinder or an active mode in which the rotating shaft is pivotally rotated by the active power.

In this case, the knee joint module may include a fastening portion coupled to the lower leg prosthesis of the patient with femoral amputation, and a coupling portion coupled to both ends of the rotation shaft.

In this case, the rotary hydraulic cylinder may include a cylinder body formed in a cylindrical shape, and having a space formed to accommodate a fluid therein; the rotation shaft passing through the inside of the cylinder body; a blade extending from the rotation shaft in a radial direction of the cylinder body to partition a space formed inside the cylinder body into a plurality of chambers; and a flow path control member for controlling the movement of fluid between the plurality of chambers, wherein the control module may selectively operate the knee prosthesis apparatus in any one of the passive mode and the active mode by controlling the driving of the flow path control member.

In this case, the blade may be formed to be in close contact with the inner peripheral surface and the inner surface of the cylinder body such that the movement of fluid between the plurality of chambers is made only through the flow path control member.

In this case, in the blade, the contact surface contacting the inner peripheral surface and the inner surface of the cylinder body may be formed of a rubber material.

In this case, a first pulley to which the driving cable is fastened may be formed along the outer peripheral surface of the cylinder body.

In this case, the flow path control member may include a housing disposed in a space formed inside the cylinder body and formed with a flow path through which the fluid can move; and a flow rate control valve formed inside the housing, which is capable of controlling the degree of the movement of fluid moving in a direction from one chamber to the other chamber among the plurality of chambers.

In this case, the flow rate control valve may include a first opening formed in a part of a nozzle insertion space formed inside the housing, through which the fluid can move; and an inner nozzle inserted into the nozzle insertion space and having a second opening corresponding to the first opening formed on an outer peripheral surface so as to adjust the degree of opening and closing of the first opening according to rotation.

In this case, the flow rate control valve may further include a valve motor connected to the inner nozzle to rotate the inner nozzle.

In this case, the inner nozzle and the valve motor may be connected through a bevel gear.

In this case, the flow rate control valve may include a first flow rate control valve for controlling the degree of the movement of fluid moving in a direction from a first chamber to a second chamber among the plurality of chambers; and a second flow rate control valve for controlling the degree of the movement of fluid moving in a direction from the second chamber to the first chamber in order to pivotally rotate the rotation shaft in both directions such that the knee joint module can perform both flexion and extension movements.

In this case, when the movement of fluid between the plurality of chambers is blocked by the flow rate control valve, the pivot rotation of the blade may be limited, and as the entire cylinder body is rotated by the driving cable, the rotation shaft may be pivotally rotated at the same time to operate the active mode.

In this case, when the movement of fluid between the plurality of chambers is allowed by the flow rate control valve, as the blade is pivotally rotated by the hydraulic pressure, the rotation shaft may be pivotally rotated at the same time to operate the passive mode.

In this case, the control module may control the damping speed of the knee joint module by controlling the degree of opening of the flow rate control valve by determining a walking environment in real time, based on walking information obtained through a sensor mounted on the hybrid-type knee prosthesis apparatus.

In this case, the flow path control member may be provided with a check valve for limiting the movement direction of the fluid into one direction inside the housing.

In this case, a rotor may be disposed outside the electric motor, and a second pulley to which the driving cable is fastened may be formed along an outer peripheral surface of the rotor.

In this case, a rotor may be disposed inside the electric motor, and a second pulley to which the driving cable is fastened may be formed along an outer peripheral surface of a driving shaft connected to the rotor.

In this case, when the knee prosthesis apparatus is controlled in the passive mode, the driving of the electric motor may be stopped by a brake installed therein.

### [Advantageous Effects]

The hybrid-type knee prosthesis apparatus having a rotary hydraulic cylinder according to an exemplary embodiment of the present invention can appropriately provide a prosthesis wearer with the force required for daily life by using passive power generated by a rotary hydraulic cylinder during normally walking on flat ground and walking on a slope at a predetermined angle or less, or by using active power generated by an electric motor during movement that requires a large force, such as walking on a slope at a predetermined angle or more or climbing stairs.

Since the hybrid-type knee prosthesis apparatus having a rotary hydraulic cylinder according to an exemplary embodiment of the present invention does not use active power when using passive power, there is an advantage in that energy loss is small and it can be used for a long period of time. In particular, in the case of walking on flat ground that accounts for most of the walking situations, walking on a slope at a predetermined angle or less and walking down stairs, it is possible to walk using only passive power, and thus, the energy saving effect is great.

By controlling the flow path in the rotary hydraulic cylinder through a unique flow path controlling member, the hybrid-type knee prosthesis apparatus having a rotary hydraulic cylinder according to an exemplary embodiment of the present invention is capable of achieving mode switching between the passive mode and the active mode very quickly and easily without the introduction of a separate clutch module. Through this, since the unique rotary hydraulic cylinder itself can excellently perform a clutch function for power conversion, it is possible to achieve the simplification of a device structure and the minimization of size.

By controlling the damping speed and size of a knee joint in real time through precise hydraulic control in the rotary hydraulic cylinder when operated in the passive mode, the hybrid-type knee prosthesis apparatus according to an exemplary embodiment of the present invention allows a wearer of the prosthesis apparatus to maintain stable walking even in various walking environments.

### [Description of Drawings]

FIG. 1 is a diagram showing the knee angle and knee power in stance and swing phases when a person walks. The solid line represents the knee angle of a normal person, and the dotted line represents the knee angle of a disabled person with amputation during walking. Unlike the normal person with the blue solid line, the disabled person with amputation with the red dotted line walks with almost no stance phase flexion due to anxiety about falling.
FIG. 2 is a diagram showing a state in which the hybrid-type knee prosthesis apparatus according to an exemplary embodiment of the present invention is mounted on the knee portion of a patient with femoral amputation. In this case, FIG. 2(a) illustrates that the prosthesis apparatus is worn above the knee because the affected part of the patient with femoral amputation is short, and conversely, FIG. 2(b) illustrates that the prosthesis apparatus is worn below the knee because the affected part is long.
FIGS. 3 and 4 are diagrams showing different viewing angles of a high-torque prosthesis apparatus among hybrid-type knee prosthesis apparatuses according to an exemplary embodiment of the present invention.
FIG. 5 is an exploded view showing the hybrid-type knee prosthesis apparatus of FIGS. 3 and 4.
FIG. 6 is a diagram showing a rotary hydraulic cylinder of the hybrid-type knee prosthesis apparatus according to an exemplary embodiment of the present invention.
FIG. 7 is a diagram for describing the operation of a flow rate control valve of the hybrid-type knee prosthesis apparatus according to an exemplary embodiment of the present invention.
FIG. 8 is a diagram for describing another example of a flow rate control valve of the hybrid-type knee prosthesis apparatus according to an exemplary embodiment of the present invention.
FIG. 9 is a diagram schematically illustrating the flow of fluid in a rotary hydraulic cylinder of the hybrid-type knee prosthesis apparatus according to an exemplary embodiment of the present invention.
FIG. 10 is a diagram showing different viewing angles of a low-torque prosthesis apparatus among hybrid-type knee prosthesis apparatuses according to an exemplary embodiment of the present invention.
FIG. 11 is a diagram for describing the operations of the passive mode and the active mode of the hybrid-type knee prosthesis apparatus according to an exemplary embodiment of the present invention.
FIG. 12 is a diagram illustrating the method of controlling an operation by the control module of the hybrid-type knee prosthesis apparatus according to an exemplary embodiment of the present invention in the case of a disabled person with amputation having poor walking ability.
FIG. 13 is a diagram illustrating the method of controlling an operation by the control module of the hybrid-type knee prosthesis apparatus according to an exemplary embodiment of the present invention in the case of a disabled person with amputation having walking ability.

### [Modes of the Invention]

Hereinafter, exemplary embodiments of the present invention will be described in detail with reference to the accompanying drawings such that those of ordinary skill in the art to which the present invention pertains may easily practice the present invention. The present invention may be implemented in various different forms, and is not limited to the exemplary embodiments described herein. In the drawings, parts irrelevant to the description are omitted in order to clearly describe the present invention, and the same reference numerals are assigned to the same or similar components throughout the specification.

In the present specification, terms such as "include", "have" or the like are intended to designate that a feature, a number, a step, an operation, a component, a part or a combination thereof described in the specification exists, and these should be understood such that it does not preclude the possibility of the presence or addition of one or more other features or numbers, steps, operations, components, parts or combinations thereof.

The hybrid-type knee prosthesis apparatus having a rotary hydraulic cylinder 10 (hereinafter, `a hybrid-type knee prosthesis apparatus') according to an exemplary embodiment of the present invention is an apparatus which is mounted on the femoral region of a patient with femoral amputation to assist in walking by simulating the flexion motion or extension motion by the actual knee joint of the human body.

In this case, by providing power to a knee joint module 20 to be described below, the hybrid-type knee prosthesis apparatus 10 according to an exemplary embodiment of the present invention is a hybrid power-based apparatus that may selectively apply passive power by a rotary hydraulic cylinder 30 and active power by an electric motor 50. In this regard, the hybrid-type knee prosthesis apparatus 10 according to an exemplary embodiment of the present invention may accurately and easily perform the above-described power conversion by introducing the rotary hydraulic cylinder 30 having a unique structure. Hereinafter, the main configuration and operation of the hybrid-type knee prosthesis apparatus 10 according to an exemplary embodiment of the present invention will be described in detail.

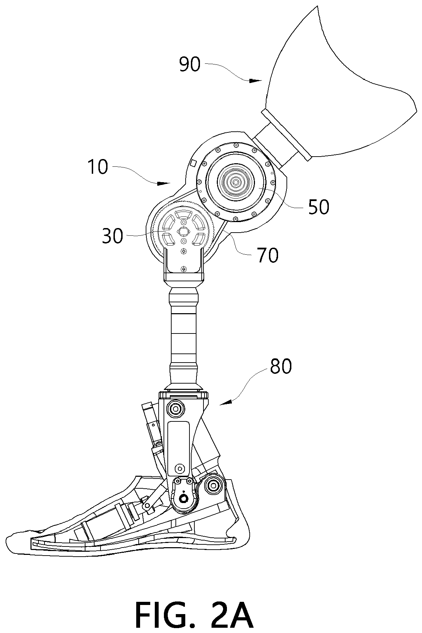

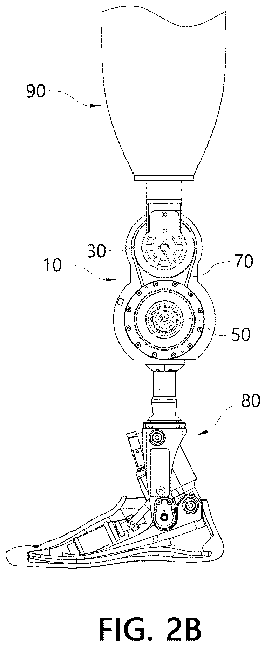

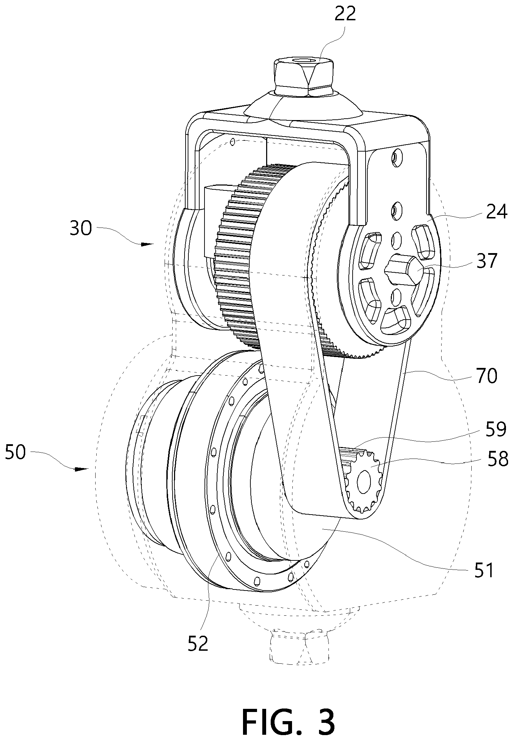

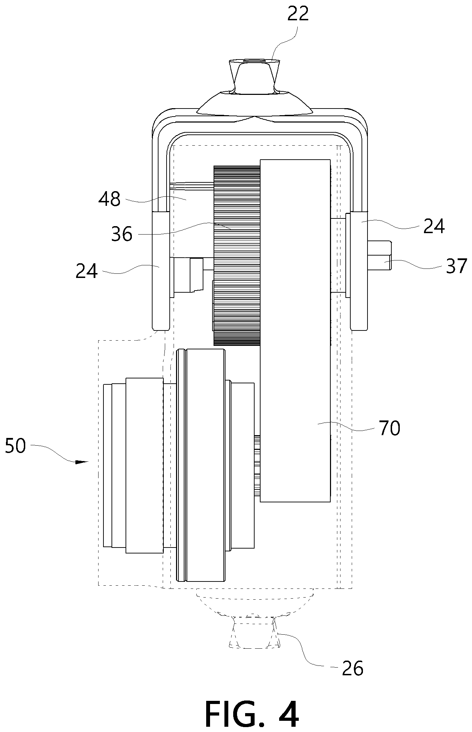

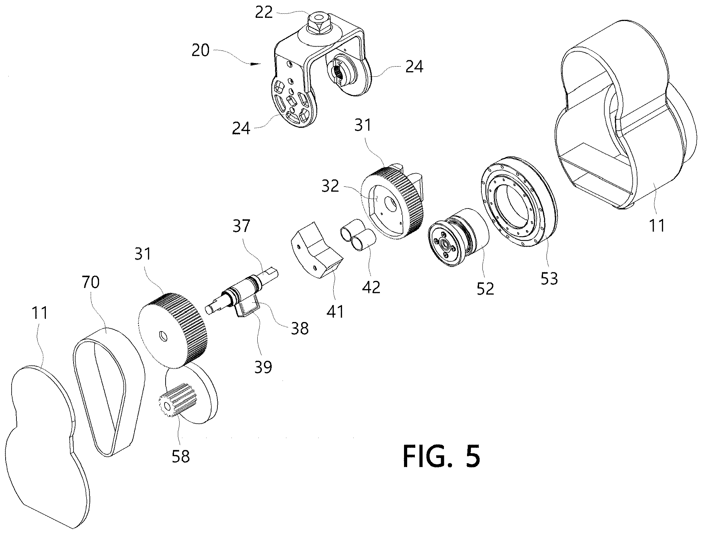

FIG. 1 is a diagram showing the knee angle and knee power in stance and swing phases when a person walks. The solid line represents the knee angle of a normal person, and the dotted line represents the knee angle of a disabled person with amputation during walking. Unlike the normal person with the blue solid line, the disabled person with amputation with the red dotted line walks with almost no stance phase flexion due to anxiety about falling. FIG. 2 is a diagram showing a state in which the hybrid-type knee prosthesis apparatus according to an exemplary embodiment of the present invention is mounted on the knee portion of a patient with femoral amputation. In this case, FIG. 2(a) illustrates that the prosthesis apparatus is worn above the knee because the affected part of the patient with femoral amputation is short, and conversely, FIG. 2(b) illustrates that the prosthesis apparatus is worn below the knee because the affected part is long. FIGS. 3 and 4 are diagrams showing different viewing angles of a high-torque prosthesis apparatus among hybrid-type knee prosthesis apparatuses according to an exemplary embodiment of the present invention. FIG. 5 is an exploded view showing the hybrid-type knee prosthesis apparatus of FIGS. 3 and 4.

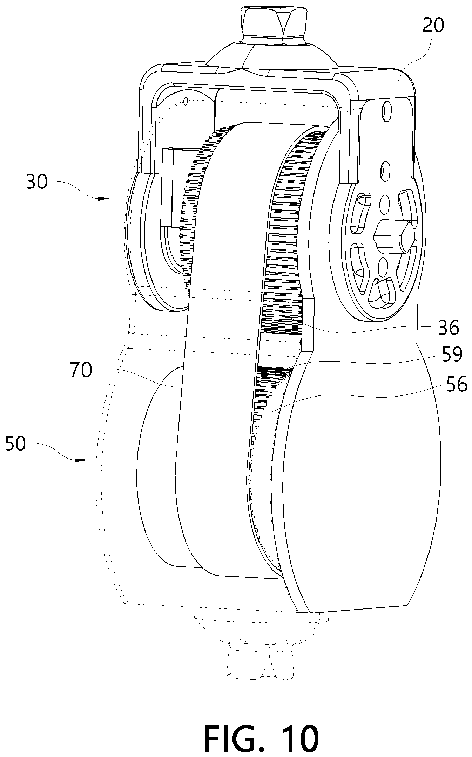

In the following description, each component of the hybrid-type prosthesis apparatus 10 according to an exemplary embodiment of the present invention will be described with reference to the high-torque hybrid-type prosthesis apparatus illustrated in FIGS. 3 to 5. The hybrid-type prosthesis apparatus 10 according to an exemplary embodiment of the present invention may include a low-torque hybrid prosthesis apparatus illustrated in FIG. 10 in addition to the high-torque type, which will be described in detail through the corresponding part.

Referring to FIGS. 2 to 5, the hybrid-type knee prosthesis apparatus 10 according to an exemplary embodiment of the present invention may include a knee joint module 20, a rotary hydraulic cylinder 30, an electric motor 50, a driving cable 70, a control module (not illustrated) and an external frame 11 that protects the above-described configurations from external forces.

First, the knee joint module 20 may have one side connected to a lower leg prosthesis 80 of a patient with femoral amputation, and the other side may be connected to a rotation shaft 37 of the rotary hydraulic cylinder 30.

In this case, the knee joint module 20 may pivotally rotate around the rotation shaft 37 by receiving power from the rotation shaft 37 of the rotary hydraulic cylinder 30. By this pivotal rotation, the hybrid-type knee prosthesis apparatus 10 according to an exemplary embodiment of the present invention may simulate the flexion motion and extension motion of the actual human knee joint.

In one exemplary embodiment of the present invention, the knee joint module 20 may include a fastening portion 22 and a coupling portion 24.

First, the fastening portion 22 is for stably fixing the hybrid-type knee prosthesis apparatus 10 according to an exemplary embodiment of the present invention to the lower leg prosthesis 80, and for example, it may be formed in a shape protruding in the direction of a fastening groove so as to be coupled to the fastening groove (not illustrated) provided in the lower leg prosthesis 80 like a pyramid head.

Next, referring to FIG. 5, the coupling portion 24 of the knee joint module 20 connects the knee joint module 20 to the rotation shaft 37 of the rotary hydraulic cylinder 30 so as to receive power from the side of the rotary hydraulic cylinder 30, and it may be coupled to both ends of the rotation shaft 37. As a non-limiting example, the coupling portion 24 may be formed of a pair of flat plate-shaped rib structures disposed parallel to each other as illustrated in FIG. 5, wherein the individual coupling portions constituting the pair of coupling portions 24 may be respectively coupled to both ends of the rotation shaft 37 of the rotary hydraulic cylinder 30. In this way, the knee joint module 20 is coupled to both ends of the rotation shaft 37 through the coupling portion 24 such that it may pivotally rotate integrally with the rotation shaft 37. Accordingly, the lower leg prosthesis 80 connected to the knee joint module 20 may also perform a pivot rotation motion by walking according to the pivot rotation.

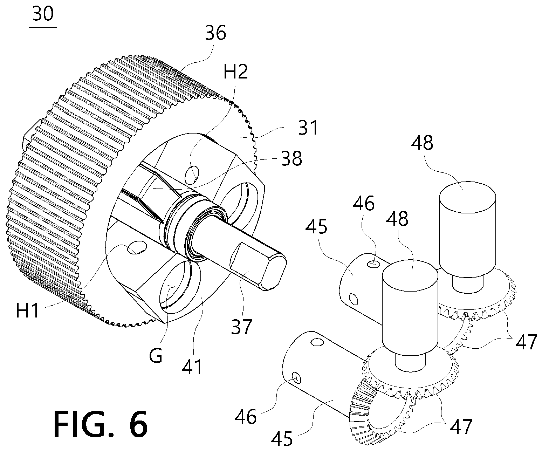

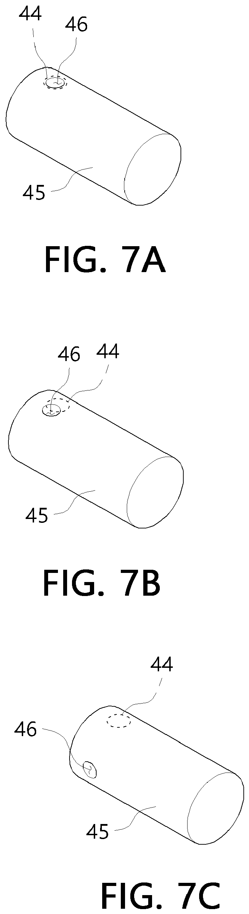

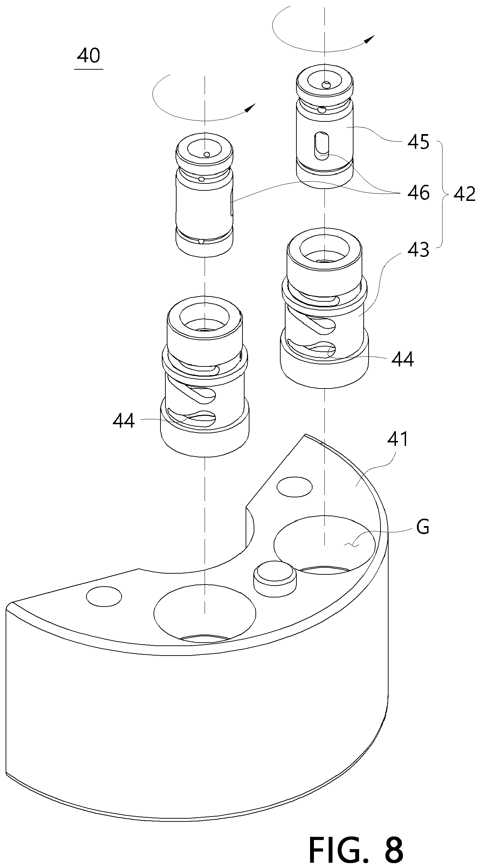

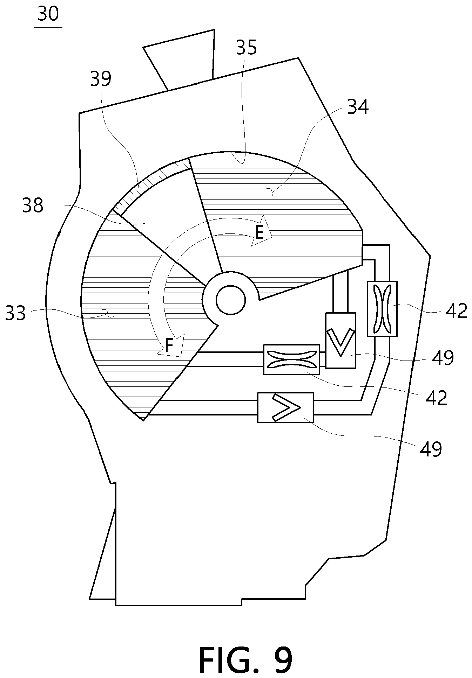

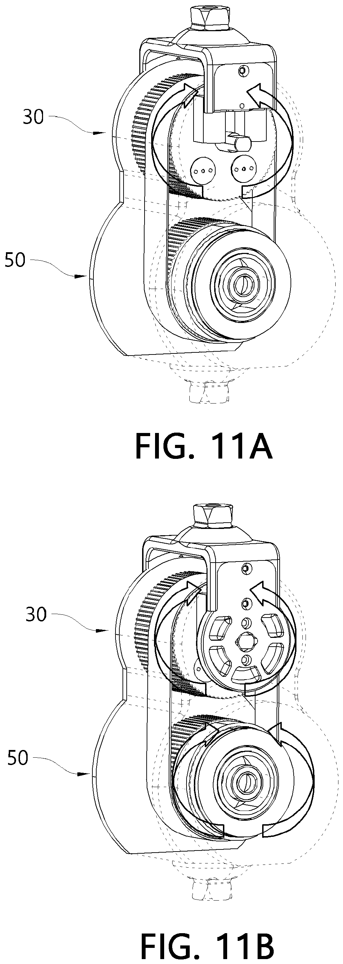

FIG. 6 is a diagram showing a rotary hydraulic cylinder of the hybrid-type knee prosthesis apparatus according to an exemplary embodiment of the present invention. FIG. 7 is a diagram for describing the operation of a flow rate control valve of the hybrid-type knee prosthesis apparatus according to an exemplary embodiment of the present invention. FIG. 8 is a diagram for describing another example of a flow rate control valve of the hybrid-type knee prosthesis apparatus according to an exemplary embodiment of the present invention. FIG. 9 is a diagram schematically illustrating the flow of fluid in a rotary hydraulic cylinder of the hybrid-type knee prosthesis apparatus according to an exemplary embodiment of the present invention. FIG. 10 is a diagram showing different viewing angles of a low-torque prosthesis apparatus among hybrid-type knee prosthesis apparatuses according to an exemplary embodiment of the present invention. FIG. 11 is a diagram for describing the operations of the passive mode and the active mode of the hybrid-type knee prosthesis apparatus according to an exemplary embodiment of the present invention.

The hybrid-type knee prosthesis apparatus 10 according to an exemplary embodiment of the present invention is operatively coupled to the above-described knee joint module 20 and may have a rotary hydraulic cylinder 30 for pivotally rotating the knee joint module 20 based on the rotation shaft 37.

More specifically, the rotary hydraulic cylinder 30 may include a cylinder body 31, a rotation shaft 37, a blade 38 and a flow path controlling member 40.

Referring to FIGS. 5 and 6, the cylinder body 31 is formed in a cylindrical shape, and a space 32 may be formed to accommodate fluid generating a hydraulic pressure therein. In addition, a first pulley 36 to which the driving cable 70 may be fastened may be formed on the outer peripheral surface of the cylinder body 31 in the circumferential direction, and the description thereof will be described in more detail through the description related to the driving cable 70.

In the center of the cylinder body 31, a rotation shaft 37 for transmitting power to the above-described knee joint module 20 may be disposed to pass through the cylinder body 31.

In one exemplary embodiment of the present invention, the rotation shaft 37 is formed to extend in the radial direction of the cylinder body 31 from the rotation shaft 37, and finally, a blade 38 in contact with the inner peripheral surface 35 of the cylinder body 31 and the inner surfaces of the cylinder body 31 on both sides may be connected. Accordingly, the blade 38 may partition a space 32 formed inside the cylinder body 31 into a plurality of chambers 33 and 34.

Meanwhile, the blade 38 may be connected to move integrally with the rotation shaft 37, through which the fluid present in the chambers 33 and 34 inside the cylinder body 31 may rotate with the rotation shaft 37 by a hydraulic pressure generated on the outer surface of the blade 38. Hereinafter, it is defined as a passive mode that by rotating the blade 38 by the hydraulic pressure generated by the rotary hydraulic cylinder 30 in this way, the rotation shaft 37 and the knee joint module connected thereto are pivotally rotated at the same time, and power used in such a passive mode is defined as passive power.

In one exemplary embodiment of the present invention, referring to FIG. 9, the blade 38 may be formed to be completely in close contact with the inner peripheral surface 35 and the inner surfaces of the cylinder body 31 on both sides. This is to secure an airtight state such that the fluid may not move between the blade 38 and the inner peripheral surface 35 and the inner surfaces of the cylinder body on both sides. As a result, in one exemplary embodiment of the present invention, the movement of fluid inside the rotary hydraulic cylinder 30 may be made only through the flow path control member 40 to be described below.

In this regard, in order for the blade 38 to be completely in contact with the inner peripheral surface 35 and the inner surfaces of the cylinder body on both sides, one end 39 of the blade 38 in contact with the inner peripheral surface 35 of the cylinder body and both sides of the blade may be formed of a rubber material. As a non-limiting example, a separate rubber packing having a shape corresponding to the shape of the blade 38 may be coupled to the blade 38.

In one exemplary embodiment of the present invention, the rotary hydraulic cylinder 30 may be provided with a flow path controlling member 40 for controlling the movement of the blade 38 by controlling the movement of fluid inside the cylinder body 31.

Referring to FIG. 9, the flow path controlling member 40 may include a housing 41 disposed between a plurality of chambers 33 and 34 inside the cylinder body 31, and a flow rate controlling valve 42 capable of controlling the degree of the movement of fluid moving in a direction from a first chamber 33 to a second chamber 35 inside the cylinder body 31 by controlling the flow path inside the housing 41.

Herein, controlling the degree of the movement of fluid may mean completely blocking, partially allowing or completely opening the flow path through which the fluid moves from the first chamber 33 to the second chamber 34. This will be described in detail in the following description.

Referring to FIGS. 6 and 7, as a specific example of the flow rate control valve 42, the flow rate control valve 42 may include a first opening 44 formed in a portion on a nozzle insertion space G formed inside the housing 41 described above, and an inner nozzle 45 inserted into the nozzle insertion space G.

In this case, through the first opening 44 formed on the nozzle insertion space G inside the housing 41, the fluid may be introduced from the inner nozzle 45 into the flow path formed inside the housing 41, or may flow out from the flow path formed inside the housing 41 towards the inner nozzle 45.

In addition, the fluid introduced into the housing 41 through the first opening 44 may move to a plurality of chambers 33 and 34 formed inside the cylinder through fluid passages H1 and H2 formed on the housing 41. Conversely, the fluid introduced into the housing 41 from the chambers 33 and 34 inside the cylinder through the fluid passages H1 and H2 of the housing 41 may flow out towards the inner nozzle 45 along the first opening 44.

In this case, a plurality of first openings 44 may be formed along the circumferential direction of the nozzle insertion space G, and as an illustrative example, any one of the first openings 44 may function as an inlet which allows the fluid to be introduced into the housing 41, and the other first opening 44 may function as an inlet through which the fluid flows out of the housing 41.

The inner nozzle 45 is a cylindrical length member having a diameter corresponding to the nozzle insertion space G, and similar to the housing 41, a flow path through which fluid may be moved may be formed therein. In this case, the flow path is formed through the inner nozzle 45 such that the fluid may move from the inside of the housing 41 to the other side of the housing 41 via the inner nozzle 45.

More specifically, a second opening 46 corresponding to the first opening 44 may be formed on the outer peripheral surface of the inner nozzle 45, and through such a second opening 46, the fluid flowing inside the inner nozzle 45 may flow out towards the flow path formed in the housing 41 via the first opening 44 formed in the housing 41 or the fluid which flows out from the first opening 44 of the housing 41 may be introduced into a flow path formed inside the inner nozzle 45. In this case, a plurality of second openings 46 may also be formed along the circumferential direction of the inner nozzle 45 like the first openings 44.

In this case, the flow rate control valve 42 may control the degree of the movement of fluid between the chambers 33 and 34 inside the cylinder body 31 according to the rotation of the inner nozzle 45. With reference to FIG. 7, the driving related to the flow rate control of the flow rate control valve 42 will be described in more detail below.

For example, as shown in (a) of FIG. 7, when the first opening 44 and the second opening 46 completely overlap, the amount of the movement of fluid between the housing 41 and the inner nozzle 45 may be maximized. In addition, as shown in (b) of FIG. 7, when the first opening 44 and the second opening 46 only partially overlap each other, the amount of the movement of fluid may be limited compared to the case where they completely overlap, and as shown in (c) of FIG. 7, when the first opening 44 and the second opening 46 do not overlap at all, it is possible to completely block the movement of fluid.

In the above, it has been described that the first opening 44 is directly formed on the nozzle insertion space G of the housing 41, and hereinafter, an exemplary embodiment in which the first opening 44 is introduced in the housing 41 using an outer nozzle 43 corresponding to the inner nozzle 45 will be described.

Specifically, referring to FIG. 8, the outer nozzle 43 is formed in a cylindrical shape having a hollow inside, and is inserted into the nozzle insertion space G of the housing 41 so as to be coupled to the housing 41

In this case, a first nozzle 44 penetrating the outer nozzle 43 may be formed on one side of the outer nozzle 43. In addition, the inner nozzle 45 having a second nozzle 46 may be inserted into the hollow formed in the center of the outer nozzle 43.

In one exemplary embodiment of the present invention, the outer nozzle 43 is fixed to the housing 41 in a coupled state, and only the inner nozzle 45 may rotate by receiving power from a valve motor 48 to be described below. As described above, as the inner nozzle 45 rotates, an area in which the first nozzle 44 at a fixed position and the rotating second nozzle 46 intersect may change, and accordingly, the amount of the movement of fluid between the inner nozzle 45 and the housing 41 may be controlled.

Meanwhile, the inner nozzle 45 may be rotated by a valve motor 48 connected to one end of the inner nozzle 45. In this case, the valve motor 48 may be an electric motor whose driving is controlled by a control module (not illustrated) to be described below and which is driven by receiving a separate power supply.

As a non-limiting example, the inner nozzle 45 and the valve motor 48 may be directly connected by a bevel gear 47 as illustrated in FIG. 7. In this way, when the inner nozzle 45 and the valve motor 48 are configured with the bevel gear 47, the valve motor 48 is disposed to be perpendicular to the rotation shaft 37 of the rotary hydraulic cylinder 30 such that there is an advantage in that it is possible to minimize the width direction thickness of the hybrid-type knee prosthesis apparatus 10 according to an exemplary embodiment of the present invention.

However, if the space in the hybrid-type knee prosthesis apparatus 10 is sufficient, the inner nozzle 45 and the valve motor 48 may be connected to a gear having a serial structure.

Meanwhile, referring to FIG. 9, in one exemplary embodiment of the present invention, the flow rate control valve 42 may separately include a first flow rate control valve 42 and a second flow rate control valve 42 in which the moving directions of the fluid are different from each other.

For example, the first flow rate control valve 42 may control the degree of the movement of fluid moving in a direction from a first chamber 33 to a second chamber 34 among a plurality of chambers in the cylinder body 31, and the second flow rate control valve 42 may control the degree of the movement of fluid moving in a direction from the second chamber 34 to the first chamber 33, that is, in a direction opposite to the fluid movement direction by the first flow rate control valve 42.

In this way, forming the first flow rate control valve 42 and the second flow rate control valve 42 separately is for pivotally rotating the rotation shaft 37 in both directions such that both of the flexion motion and the extension motion by the knee joint module 20 are possible. That is, the hybrid-type knee prosthesis apparatus 10 according to an exemplary embodiment of the present invention allows the first flow rate control valve 42 to be opened for the knee joint module 20 to perform flexion motion of bending the knee, thereby allowing the fluid to move from the first chamber 33 towards the second chamber 34, and conversely, it allows the second flow rate control valve 42 to be opened for extension motion of stretching the knee such that it is possible to induce the fluid to move from the first chamber 33 towards the second chamber 34.

In one exemplary embodiment of the present invention, it is preferable that the movement direction of the fluid in the flow path controlling member 40 is fixed in one direction as described above. To this end, the hybrid-type knee prosthesis apparatus 10 according to an exemplary embodiment of the present invention may have a check valve 49 in the flow path controlling member 40 as illustrated in FIG. 9.

Referring again to FIG. 9, the check valve 49 is formed inside the housing 41 and may be installed on a flow path controlled by the flow rate control valve 42. In this case, for the shape of the check valve 49, various check valves having known shapes such as a disk check valve, a single wafer check valve, a double wafer check valve, a swing check valve and the like may be used, and thus, the detailed descriptions thereof will be omitted.

Meanwhile, the structure of the housing 41 and the flow rate control valve 42 disclosed through the above description is only a specific example of the flow path control member 40 of the hybrid-type knee prosthesis apparatus 10 according to an exemplary embodiment of the present invention, and in addition to the exemplary embodiments disclosed herein, it should be noted that the hybrid-type knee prosthesis apparatus 10 according to an exemplary embodiment of the present invention may employ various shapes that may control the movement of fluid between a plurality of chambers 33 and 34 formed inside the rotary hydraulic cylinder 30.

The hybrid-type knee prosthesis apparatus 10 according to an exemplary embodiment of the present invention may include an electric motor 50 for providing active power to the rotary hydraulic cylinder 30.

In this case, the electric motor 50 includes a rotor 52 and a stator 53, and various types of motors that may generate a rotational force by receiving power from a separate power supply module (not illustrated) may be used. As such, the rotational force generated by the electric motor 50 may be transmitted towards the rotary hydraulic cylinder 30 via the driving cable 70.

In relation to active power transmission by the driving cable 70, referring to FIG. 3, there is a driving shaft 58 connected to the rotor 52 disposed inside the electric motor 50, and a second pulley 59 to which the driving cable 70 may be fastened may be formed along the outer peripheral surface.

In one exemplary embodiment of the present invention, the driving cable 70 may be simultaneously connected to the second pulley 59 and the first pulley 36 formed on the outer peripheral surface of the cylinder body 31, and as a result, as illustrated in FIG. 11(b), as the electric motor 50 rotates, the entire cylinder body 31 may be rotated about the rotation shaft 37 of the rotary hydraulic cylinder 30.

Meanwhile, when the active power is transmitted to the rotary hydraulic cylinder 30 by the electric motor 50, the control module may control the flow path controlling member 40 to completely block the movement of fluid between a plurality of chambers 33 and 34. As such, when the movement of fluid between the chambers 33 and 34 is blocked, the hydraulic pressure is not formed inside the chambers 33 and 34 such that the blade 38 may no longer rotate relative to the inner peripheral surface 35 of the cylinder body 31.

More specifically, as described above, the blade 38 is formed to be completely in close contact with the inner peripheral surface 35 of the cylinder body 31, and a frictional force between the cylinder body 31 and the blade 38 is generated. Therefore, when the cylinder body 31 is rotated by the above-described active power, the blade 38 also rotates integrally with the cylinder body 31. As a result, the rotation shaft 37 connected to the blade 38 also rotates, and the knee joint module 20 connected to the rotation shaft 37 may also rotate. Hereinafter, as shown in FIG. 11(b), it is defined as an active mode that the cylinder body 31 and the blade 38 are integrally rotated by the electric motor 50 such that the rotation shaft 37 and the knee joint module 20 connected thereto are also pivotally rotated at the same time, and power used in such an active mode is defined as active power.

The hybrid-type prosthesis apparatus 10 according to an exemplary embodiment of the present invention may be largely classified into two types according to the structure of the electric motor 50.

First, as illustrated in FIG. 3, there is a high-torque hybrid-type prosthesis apparatus 10 that connects a driving cable 70 through a separate driving shaft 58. In this high-torque hybrid-type prosthesis apparatus, compared to a low-torque hybrid-type prosthesis apparatus to be described below, by forming the second pulley 59 on a separate driving shaft 58, the gear ratio is increased such that there is an advantage in that higher torque power may be transmitted.

On the contrary, as illustrated in FIG. 10, when the stator is disposed inside the electric motor 50 and the rotor is disposed outside, the electric motor 50 does not form a separate driving shaft 58 for connecting the driving cable 70, and the second pulley 59 may be formed along the outer peripheral surface of the rotor 52. The hybrid-type prosthesis apparatus 10 having such a structure is defined as a low-torque hybrid-type prosthesis apparatus 10.

In this case, it is possible to save space for arranging a separate driving shaft 58 such that there is an advantage in that it is possible to form the hybrid-type knee prosthesis apparatus 10 having a more compact structure. On the other hand, compared to the high-torque prosthesis apparatus that transmits power by introducing a driving shaft 58, the gear ratio between the first pulley 36 and the second pulley 59 is reduced such that it may be somewhat disadvantageous in terms of the maximum torque that may be transmitted by the electric motor 50.

Therefore, when the degree of disability is severe and a high level of torque is required, the high-torque hybrid-type prosthesis apparatus in which the rotor 52 is disposed inside and transmits active power through the driving shaft 58 may be applied, and when the degree of disability is relatively mild, the low-torque hybrid-type prosthesis apparatus in which the rotor 52 is disposed outside to directly transmit active power by the rotor 52 without a driving shaft 58 may be selectively applied.

In addition, the hybrid-type knee prosthesis apparatus 10 according to an exemplary embodiment of the present invention may be formed such that even the same patient may replace the above-described two types of the electric motors 50 in consideration of the user's walking environment.

Through this, after the user of the hybrid-type knee prosthesis apparatus 10 according to an exemplary embodiment of the present invention considers in advance, for example, whether the day's walking environment includes a large number of situations requiring a high torque such as climbing stairs, mountain climbing or the like, or whether the installation of a compact prosthesis apparatus is advantageous because a high torque is not necessary because most of the walking will be on flat ground, the user may mount any suitable type of the electric motor 50.

In one exemplary embodiment of the present invention, when the hybrid-type knee prosthesis apparatus 10 according to an exemplary embodiment of the present invention is controlled in the passive mode, the electric motor 50 may be controlled such that the driving is stopped by a brake installed therein, for example, an electronic brake. This is to prevent the desired power from being transmitted to the knee joint module 20 due to overlap or collision between the passive power and the active power. The driving control of such an electric motor may be performed by a control module to be described below.

The hybrid-type knee prosthesis apparatus 10 according to an exemplary embodiment of the present invention may include a control module (not illustrated) for controlling the above-described configurations such that the knee prosthesis apparatus 10 operates in any one of the passive mode and the active mode.

Specifically, the control module may control the driving of the flow path controlling member 40 and the electric motor 50 together to switch between the passive mode and the active mode.

First, in order to execute the passive mode, the control module may control the driving of the flow path controlling member 40 to open the flow path between the plurality of chambers 33 and 34 in the cylinder body 31 so as to enable the movement of fluid. Further, in order to block the transmission of active power by the electric motor 50, a brake in the electric motor 50 may be activated to stop the driving of the electric motor 50. As described above, the blocking of active power in the passive mode is to prevent collision with the passive power.

In this case, the control module may control the damping speed of the knee joint module 20 by passive power by varying the degree of opening of the flow path by the flow path controlling member 40. For example, when the degree of opening of the flow path is large, the damping speed of the knee joint module 20 may be fast, and conversely, when the degree of opening of the flow path is small, the damping speed may be slow.

Such damping speed control may be performed by sensing the user's walking environment or condition in real time. For example, when the user walks slowly on a flat road, the damping speed may be slowed for a natural gait, and when the user walks at a brisk pace, the damping speed may be quickly changed accordingly to provide a stable gait.

In this case, the detection of the walking environment or condition may be performed by a sensor module (not illustrated) separately installed in the hybrid-type knee prosthesis apparatus 10 according to an exemplary embodiment of the present invention, wherein the sensor module may detect the knee angle, angular velocity and the like

Next, in order to execute the active mode, the control module may block the flow path between the plurality of chambers 33 and 34 through the flow path controlling member 40 in order to suppress the generation of hydraulic pressure by the fluid inside the cylinder body 31. In addition, by activating the driving of the electric motor 50, active power may be transmitted to the rotary hydraulic cylinder 30 through the driving cable 70.

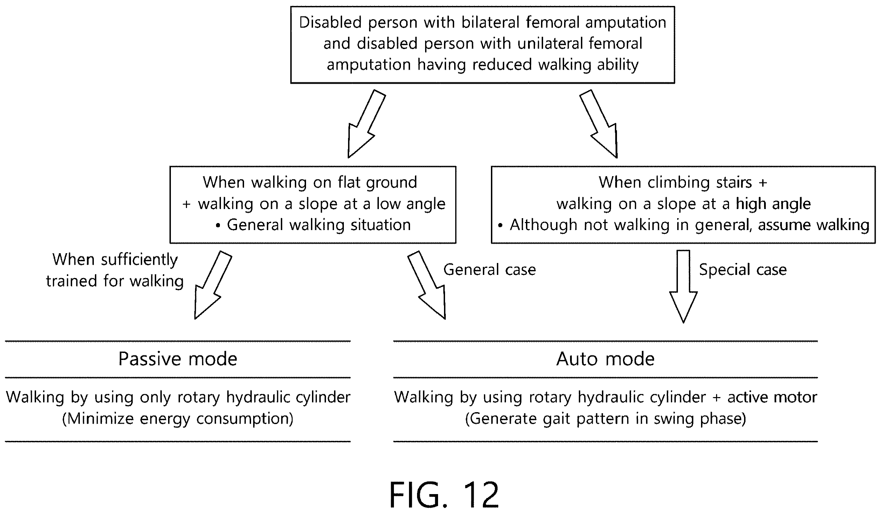

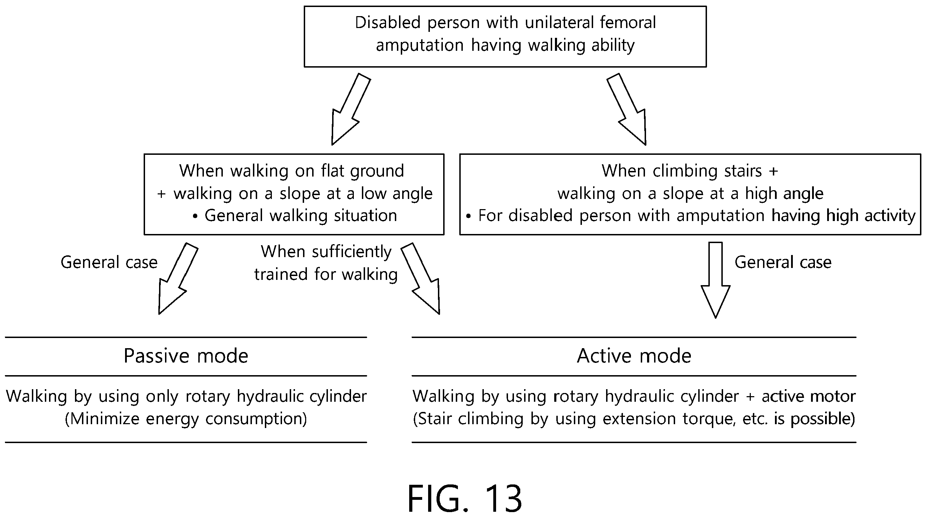

FIG. 12 is a diagram illustrating the method of controlling an operation by the control module of the hybrid-type knee prosthesis apparatus according to an exemplary embodiment of the present invention in the case of a disabled person with amputation having poor walking ability. FIG. 13 is a diagram illustrating the method of controlling an operation by the control module of the hybrid-type knee prosthesis apparatus according to an exemplary embodiment of the present invention in the case of a disabled person with amputation having walking ability.

Hereinafter, cases in which the control module of the hybrid-type knee prosthesis apparatus 10 according to an exemplary embodiment of the present invention selectively applies the passive mode and the active mode, respectively, will be described in more detail in relation to the user's walking condition.

First, referring to FIG. 12, for example, cases in which a wearer with walking ability below a certain level, such as a disabled person who has both thighs amputated and a disabled person with unilateral femoral amputation with poor walking ability, uses the hybrid-type knee prosthesis apparatus 10 will be described.

In the case of a general walking situation that occupies a significant part of daily life, such as walking on flat ground and walking on a low slope at a predetermined angle or less, if the wearer has performed sufficient gait training while wearing the hybrid-type knee prosthesis apparatus 10, the hybrid-type knee prosthesis apparatus 10 may be controlled in the passive mode using only a rotary hydraulic cylinder. In this case, since the wearer has performed sufficient gait training, a certain portion of the force may be shared during the swing phase, and therefore, it is possible to sufficiently simulate the swing motion of the knee joint from the swing phase to the stance phase only with the damping force by the rotary hydraulic cylinder. Through this, there is an advantage in that the wearing time may be improved by minimizing the energy consumption of the hybrid-type knee prosthesis apparatus 10.

However, in the case of a wearer who has not performed sufficient gait training, even in a general walking situation, if only the passive mode is used, walking itself may be difficult, and thus, it may be controlled in an `auto mode' in which the passive mode by the rotary hydraulic cylinder and the active mode by the electric motor are automatically changed.

Meanwhile, in the case of a walking situation that requires a lot of force, such as climbing stairs or walking on a slope at a high angle, rather than a general gait, since the hybrid-type prosthesis apparatus 10 may be operated only in the passive mode, it may be controlled in the auto mode regardless of whether gait training has been performed.

Next, referring to FIG. 13, in the case of a disabled person with unilateral femoral amputation having walking ability, the control method of the control module provided in the hybrid-type prosthesis apparatus 10 will be described.

For a wearer with some walking ability, energy consumption may be minimized by controlling the hybrid-type prosthesis apparatus 10 to use only the passive mode in a general walking environment such as flat ground. In this case, since the wearer has muscle strength which is greater than or equal to a predetermined level, even a wearer without gait training may sufficiently walk in the passive mode alone.

However, even for a wearer who has performed walking ability, it is difficult to sufficiently walk only in the passive mode of the hybrid-type prosthesis apparatus 10 in the case of climbing stairs and walking on a slope at a high angle. Accordingly, in this case, the hybrid-type prosthesis apparatus 10 may be controlled such that the passive mode by the rotary hydraulic cylinder and the active mode by the electric motor are automatically changed.

However, in this case, when the difference from the `auto mode' of the wearer without walking ability described above is reviewed, since the auto mode provided to the wearer without walking ability is a general walking situation, the electric motor generates only the swing motion pattern of the knee joint only in the swing phase and operates as a brake in the stance phase. However, in the case of a wearer having walking ability, it may provide a torque in an extension direction that lifts the body during the stance phase to assist more activities, and during the swing phase, there is a difference in that it is possible to generate a gait pattern in the same way as in the auto mode.

As such, the hybrid-type prosthesis apparatus 10 according to an exemplary embodiment of the present invention may freely change the passive mode by the rotary hydraulic cylinder and the active mode by the electric motor at each step of walking by the pedestrian without a separate clutch module. This is because the flow path control by the rotary hydraulic cylinder 30 provides a clutch (power conversion) function and a braking function. In addition, by controlling the degree of the movement of fluid by the rotary hydraulic cylinder 30, it is possible to precisely control the damping speed and degree of the rotary hydraulic cylinder 30. That is, the rotary hydraulic cylinder 30 of the hybrid-type prosthesis apparatus 10 according to an exemplary embodiment of the present invention has an advantage of having various functions such as a clutch function, a damping control function, a braking function and the like.

As reviewed above, since the hybrid-type knee prosthesis apparatus 10 according to an exemplary embodiment of the present invention provides active power by specifying a section in which the active driving mode is to be operated, for example, a section in which the active mode is activated during walking on a slope, and a section in which the active mode is operated during climbing stairs, the energy consumption section may be minimized while providing a pedestrian with an appropriate force required for the pedestrian in the corresponding section, and thus, the hybrid-type prosthesis apparatus may be efficiently used for a long period of time.

In this way, the hybrid-type prosthesis apparatus that blocks the operation of the passive mode by the rotary hydraulic cylinder 30 and efficiently operates the electric motor 50 only when an active driving is required uses energy efficiently, and thus, it is possible to reduce the size of the battery which is mounted on the prosthesis apparatus. Accordingly, it is possible to reduce the weight of the hybrid-type prosthesis apparatus, thereby providing a compact hybrid-type prosthesis apparatus.

In addition, even when the active mode is to be operated, the hybrid-type prosthesis apparatus according to an exemplary embodiment of the present invention provides a force by operating the active mode only in at least some sections in the stance phase section, and uses passive power by the rotary hydraulic cylinder in the swing phase section, thereby maximizing battery life by minimizing battery use.

In particular, by controlling a hydraulic pressure in the rotary hydraulic cylinder 30 through the unique flow path controlling member 40, the hybrid-type knee prosthesis apparatus 10 according to an exemplary embodiment of the present invention may easily achieve mode switching between the passive mode and the active mode without the introduction of a separate clutch module. That is, the rotary hydraulic cylinder 30 itself may excellently perform a clutch function for power conversion, thereby simplifying the structure of the apparatus and minimizing the size.

In addition, by controlling the damping speed in real time through the flow path control inside the rotary hydraulic cylinder 30, the hybrid-type knee prosthesis apparatus 10 according to an exemplary embodiment of the present invention allows a wearer of the prosthesis apparatus to maintain stable walking even in various walking environments.

## Claims

1. A hybrid-type knee prosthesis apparatus (10) having a multifunctional rotary hydraulic cylinder (30) which is mounted on the knee portion of a patient with femoral amputation to assist in walking, comprising:
a knee joint module (20) connected to a lower leg prosthesis (80) of the patient with femoral amputation;
a rotary hydraulic cylinder (30) operatively coupled to the knee joint module (20) and pivotally rotating the knee joint module (20) by pivot rotation of a rotation shaft (37);
an electric motor (50) for providing active power to the rotary hydraulic cylinder (30); and
a control module for controlling to operate in any one mode of a passive mode in which the rotary shaft provided in the rotary hydraulic cylinder (30) is pivotally rotated by a hydraulic pressure formed inside the rotary hydraulic cylinder (30) or an active mode in which the rotating shaft is pivotally rotated by the active power,
**characterized in that** the hybrid-type knee prosthesis apparatus (10) comprises a driving cable for operatively connecting the rotary hydraulic cylinder (30) and the electric motor (50) to each other.

2. The hybrid-type knee prosthesis apparatus (10) of claim 1, wherein the knee joint module (20) comprises a fastening portion (22) coupled to the lower leg prosthesis (80) of the patient with femoral amputation, and a coupling portion (24) coupled to both ends of the rotation shaft (37).

3. The hybrid-type knee prosthesis apparatus (10) of claim 1, wherein the rotary hydraulic cylinder (30) comprises:
a cylinder body (31) formed in a cylindrical shape, and having a space (32) formed to accommodate a fluid therein;
the rotation shaft (37) passing through the inside of the cylinder body (31);
a blade extending from the rotation shaft (37) in a radial direction of the cylinder body (31) to partition a space (32) formed inside the cylinder body (31) into a plurality of chambers (33, 34); and
a flow path control member (40) for controlling the movement of fluid between the plurality of chambers (33, 34),
wherein the control module selectively operates the knee prosthesis apparatus (10) in any one of the passive mode and the active mode by controlling the driving of the flow path control member (40).

4. The hybrid-type knee prosthesis apparatus (10) of claim 3, wherein the blade is formed to be in close contact with the inner peripheral surface and the inner surface of the cylinder body (31) such that the movement of fluid between the plurality of chambers (33, 34) is made only through the flow path control member (40).

5. The hybrid-type knee prosthesis apparatus (10) of claim 4, wherein in the blade, the contact surface contacting the inner peripheral surface and the inner surface of the cylinder body (31) is formed of a rubber material.

6. The hybrid-type knee prosthesis apparatus (10) of claim 3, wherein a first pulley to which the driving cable is fastened is formed along the outer peripheral surface of the cylinder body (31).

7. The hybrid-type knee prosthesis apparatus (10) of claim 3, wherein the flow path control member (40) comprises:
a housing disposed in a space formed inside the cylinder body (31) and formed with a flow path through which the fluid can move; and
a flow rate control valve formed inside the housing, which is capable of controlling the degree of the movement of fluid moving in a direction from one chamber to the other chamber among the plurality of chambers (33, 34).

8. The hybrid-type knee prosthesis apparatus (10) of claim 7, wherein the flow rate control valve comprises:
a first opening formed in a part of a nozzle insertion space formed inside the housing, through which the fluid can move; and
an inner nozzle inserted into the nozzle insertion space and having a second opening corresponding to the first opening formed on an outer peripheral surface so as to adjust the degree of opening and closing of the first opening according to rotation.

9. The hybrid-type knee prosthesis apparatus (10) of claim 8, wherein the flow rate control valve further comprises a valve motor connected to the inner nozzle to rotate the inner nozzle.

10. The hybrid-type knee prosthesis apparatus (10) of claim 9, wherein the inner nozzle and the valve motor are connected through a bevel gear.

11. The hybrid-type knee prosthesis apparatus (10) of claim 7, wherein the flow rate control valve comprises:
a first flow rate control valve for controlling the degree of the movement of fluid moving in a direction from a first chamber to a second chamber among the plurality of chambers (33, 34); and
a second flow rate control valve for controlling the degree of the movement of fluid moving in a direction from the second chamber to the first chamber, in order to pivotally rotate the rotation shaft (37) in both directions such that the knee joint module (20) can perform both flexion and extension movements.

12. The hybrid-type knee prosthesis apparatus (10) of claim 7, wherein when the movement of fluid between the plurality of chambers (33, 34) is blocked by the flow rate control valve, the pivot rotation of the blade is limited, and as the entire cylinder body (31) is rotated by the driving cable, the rotation shaft (37) is pivotally rotated at the same time to operate the active mode.

13. The hybrid-type knee prosthesis apparatus (10) of claim 7, wherein when the movement of fluid between the plurality of chambers (33, 34) is allowed by the flow rate control valve, as the blade is pivotally rotated by the hydraulic pressure, the rotation shaft (37) is pivotally rotated at the same time to operate the passive mode.

14. The hybrid-type knee prosthesis apparatus (10) of claim 13, wherein the control module controls the damping speed of the knee joint module (20) by controlling the degree of opening of the flow rate control valve by determining a walking environment in real time, based on walking information obtained through a sensor mounted on the hybrid-type knee prosthesis apparatus (10).

15. The hybrid-type knee prosthesis apparatus (10) of claim 7, wherein the flow path control member (40) is provided with a check valve for limiting the movement direction of the fluid into one direction inside the housing.

16. The hybrid-type knee prosthesis apparatus (10) of claim 1, wherein a rotor is disposed outside the electric motor (50), and a second pulley to which the driving cable is fastened is formed along an outer peripheral surface of the rotor.

17. The hybrid-type knee prosthesis apparatus (10) of claim 1, wherein a rotor is disposed inside the electric motor (50), and a second pulley to which the driving cable is fastened is formed along an outer peripheral surface of a driving shaft connected to the rotor.

18. The hybrid-type knee prosthesis apparatus (10) of claim 1, wherein when the knee prosthesis apparatus is controlled in the passive mode, the driving of the electric motor (50) is stopped by a brake installed therein.

## Patentansprüche

1. Hybrid-Knieprothesenvorrichtung (10) mit einem multifunktionalen rotierbaren Hydraulikzylinder (30), der am Kniebereich eines Patienten mit Oberschenkelamputation montiert ist, um das Gehen zu unterstützen, umfassend: ein Kniegelenkmodul (20), das mit einer Unterschenkelprothese (80) des Patienten mit Oberschenkelamputation verbunden ist;
einen rotierbaren Hydraulikzylinder (30), der funktionsfähig mit dem Kniegelenkmodul (20) gekoppelt ist und das Kniegelenkmodul (20) durch die Drehbewegung einer Drehwelle (37) schwenkend dreht;
einen Elektromotor (50) zur Bereitstellung von aktiver Leistung für den rotierenden Hydraulikzylinder (30); und
ein Steuermodul zum Steuern des Betriebs in einem beliebigen Modus eines passiven Modus, in dem die in dem rotierbaren Hydraulikzylinder (30) vorgesehene Drehwelle durch einen im Inneren des rotierbaren Hydraulikzylinders (30) gebildeten hydraulischen Druck schwenkbar gedreht wird, oder eines aktiven Modus, in dem die Drehwelle durch die aktive Kraft schwenkbar gedreht wird,
**dadurch gekennzeichnet, dass** die Hybrid-Knieprothesenvorrichtung (10) ein Antriebskabel umfasst, um den rotierbaren Hydraulikzylinder (30) und den Elektromotor (50) funktionell miteinander zu verbinden.

2. Hybrid-Knieprothesenvorrichtung (10) nach Anspruch 1, wobei das Kniegelenkmodul (20) einen Befestigungsabschnitt (22) umfasst, der mit der Unterschenkelprothese (80) des Patienten mit Oberschenkelamputation verbunden ist, und einen Koppelabschnitt (24), der mit beiden Enden der Drehwelle (37) verbunden ist.

3. Die Hybrid-Knieprothesenvorrichtung (10) nach Anspruch 1, wobei der rotierbare Hydraulikzylinder (30) umfasst:
einen Zylinderkörper (31), der in zylindrischer Form ausgebildet ist und einen Raum (32) aufweist, der zur Aufnahme einer Flüssigkeit darin gebildet ist; die Drehwelle (37), die durch das Innere des Zylinderkörpers (31) verläuft;
eine Klinge, die sich von der Drehwelle (37) in radialer Richtung des Zylinderkörpers (31) erstreckt, um den im Inneren des Zylinderkörpers (31) gebildeten Raum (32) in eine Vielzahl von Kammern (33, 34) zu unterteilen; und ein Strömungsweg-Steuerelement (40) zur Steuerung der Bewegung der Flüssigkeit zwischen den verschiedenen Kammern (33, 34),
wobei das Steuermodul die Knieprothesenvorrichtung (10) selektiv in einem der beiden Modi, dem passiven Modus und dem aktiven Modus, betreibt, indem es die Ansteuerung des Strömungsweg-Steuerelements (40) steuert.

4. Hybrid-Knieprothesenvorrichtung (10) nach Anspruch 3, wobei die Klinge so ausgebildet ist, dass sie in engem Kontakt mit der inneren Umfangsfläche und der inneren Oberfläche des Zylinderkörpers (31) steht, sodass die Bewegung der Flüssigkeit zwischen den verschiedenen Kammern (33, 34) nur durch das Strömungsweg-Steuerelement (40) erfolgt.

5. Hybrid-Knieprothesenvorrichtung (10) nach Anspruch 4, wobei die Kontaktfläche der Klinge, die mit der inneren Umfangsfläche und der inneren Oberfläche des Zylinderkörpers (31) in Kontakt steht, aus einem Gummimaterial besteht.

6. Hybrid-Knieprothesenvorrichtung (10) nach Anspruch 3, wobei eine erste Riemenscheibe, an der das Antriebskabel befestigt ist, entlang der äußeren Umfangsfläche des Zylinderkörpers (31) ausgebildet ist.

7. Hybrid-Knieprothesenvorrichtung (10) nach Anspruch 3, wobei das Strömungsweg-Steuerelement (40) umfasst:
ein Gehäuse, das in einem Raum innerhalb des Zylinderkörpers (31) angeordnet ist und mit einem Strömungsweg versehen ist, durch den die Flüssigkeit sich bewegen kann; und
ein Durchflussregelventil, das innerhalb des Gehäuses ausgebildet ist und in der Lage ist, das Maß der Bewegung der Flüssigkeit, die in eine Richtung von einer Kammer zur anderen Kammer unter den verschiedenen Kammern (33, 34) strömt, zu steuern.

8. Hybrid-Knieprothesenvorrichtung (10) nach Anspruch 7, wobei das Durchflussregelventil umfasst:
eine erste Öffnung, die in einem Teil eines Düseneinführraums im Inneren des Gehäuses ausgebildet ist, durch die die Flüssigkeit strömen kann; und
eine Innendüse, die in den Düseneinführraum eingesetzt ist und eine zweite Öffnung aufweist, die der ersten Öffnung an der äußeren Umfangsfläche entspricht, um den Öffnungs- und Schließgrad der ersten Öffnung durch Drehung zu regulieren.

9. Hybrid-Knieprothesenvorrichtung (10) nach Anspruch 8, wobei das Durchflussregelventil ferner einen Ventilmotor umfasst, der mit der Innendüse verbunden ist, um die Innendüse zu rotieren.

10. Hybrid-Knieprothesenvorrichtung (10) nach Anspruch 9, wobei die Innendüse und der Ventilmotor über ein Kegelrad verbunden sind.

11. Hybrid-Knieprothesenvorrichtung (10) nach Anspruch 7, wobei das Durchflussregelventil umfasst:
ein erstes Durchflussregelventil zur Steuerung des Bewegungsgrades der Flüssigkeit in eine Richtung von einer ersten Kammer zu einer zweiten Kammer unter den verschiedenen Kammern (33, 34); und
ein zweites Durchflussregelventil zur Steuerung des Bewegungsgrades der Flüssigkeit in eine Richtung von der zweiten Kammer zur ersten Kammer, um die Drehwelle (37) in beide Richtungen schwenkbar zu rotieren, sodass das Kniegelenkmodul (20) sowohl Beuge- als auch Streckbewegungen ausführen kann.

12. Hybrid-Knieprothesenvorrichtung (10) nach Anspruch 7, wobei, wenn die Bewegung der Flüssigkeit zwischen den verschiedenen Kammern (33, 34) durch das Durchflussregelventil blockiert wird, die Schwenkbewegung der Klinge begrenzt ist und, da der gesamte Zylinderkörper (31) durch das Antriebskabel gedreht wird, die Drehwelle (37) gleichzeitig schwenkbar rotiert wird, um den aktiven Modus zu betreiben.

13. Hybrid-Knieprothesenvorrichtung (10) nach Anspruch 7, wobei, wenn die Bewegung der Flüssigkeit zwischen den verschiedenen Kammern (33, 34) durch das Durchflussregelventil erlaubt wird, die Klinge durch den Hydraulikdruck schwenkbar rotiert wird und die Drehwelle (37) gleichzeitig schwenkbar rotiert wird, um den passiven Modus zu betreiben.

14. Hybrid-Knieprothesenvorrichtung (10) nach Anspruch 13, wobei das Steuermodul die Dämpfungsgeschwindigkeit des Kniegelenkmoduls (20) steuert, indem es den Öffnungsgrad des Durchflussregelventils steuert, indem es die Gehbedingungen in Echtzeit auf der Grundlage von Gehinformationen bestimmt, die durch einen an der Hybrid-Knieprothesenvorrichtung (10) montierten Sensor erhalten wurden.

15. Hybrid-Knieprothesenvorrichtung (10) nach Anspruch 7, wobei das Strömungsweg-Steuerelement (40) mit einem Rückschlagventil versehen ist, das die Bewegungsrichtung der Flüssigkeit im Inneren des Gehäuses auf eine Richtung begrenzt.

16. Hybrid-Knieprothesenvorrichtung (10) nach Anspruch 1, wobei ein Rotor außerhalb des Elektromotors (50) angeordnet ist und eine zweite Riemenscheibe, an der das Antriebskabel befestigt ist, entlang der äußeren Umfangsfläche des Rotors ausgebildet ist.

17. Hybrid-Knieprothesenvorrichtung (10) nach Anspruch 1, wobei ein Rotor innerhalb des Elektromotors (50) angeordnet ist und eine zweite Riemenscheibe, an der das Antriebskabel befestigt ist, entlang der äußeren Umfangsfläche einer mit dem Rotor verbundenen Antriebswelle ausgebildet ist.

18. Hybrid-Knieprothesenvorrichtung (10) nach Anspruch 1, wobei, wenn die Knieprothesenvorrichtung im passiven Modus gesteuert wird, der Antrieb des Elektromotors (50) durch eine darin installierte Bremse gestoppt wird.

## Revendications

1. Appareil de prothèse de genou de type hybride (10) comportant un cylindre hydraulique rotatif multifonctionnel (30) qui est monté sur la partie du genou d'un patient souffrant d'une amputation fémorale pour l'aider à marcher, comprenant :
un module d'articulation du genou (20) relié à une prothèse de jambe inférieure (80) du patient ayant subi une amputation fémorale ;
un cylindre hydraulique rotatif multifonctionnel (30) couplé de manière opérationnelle au module d'articulation du genou (20) et faisant pivoter le module d'articulation du genou (20) par rotation d'un arbre de rotation (37) ;
un moteur électrique (50) pour fournir une puissance active au cylindre hydraulique rotatif multifonctionnel (30) ; et
un module de commande pour contrôler le fonctionnement dans l'un des modes suivants : un mode passif dans lequel l'arbre rotatif fourni dans le cylindre hydraulique rotatif multifonctionnel (30) est pivoté par une pression hydraulique formée à l'intérieur du cylindre hydraulique rotatif multifonctionnel (30) ou un mode actif dans lequel l'arbre rotatif est pivoté par la puissance active,
**caractérisé par le fait que** l'appareil de prothèse de genou de type hybride (10) comprend un câble d'entraînement pour relier de manière opérationnelle le cylindre hydraulique rotatif multifonctionnel (30) et le moteur électrique (50) l'un à l'autre.

2. L'appareil de prothèse de genou de type hybride (10) de la revendication 1, dans lequel le module d'articulation du genou (20) comprend une partie de fixation (22) couplée à la prothèse de jambe inférieure (80) du patient ayant subi une amputation fémorale, et une partie de couplage (24) couplée aux deux extrémités de l'arbre de rotation (37).

3. L'appareil de prothèse de genou de type hybride (10) de la revendication 1, dans lequel le cylindre hydraulique rotatif multifonctionnel (30) comprend :
un corps de cylindre (31) de forme cylindrique, doté d'un espace (32) destiné à accueillir un fluide ;
l'arbre de rotation (37) traversant l'intérieur du corps de cylindre (31) ;
une lame s'étendant à partir de l'arbre de rotation (37) dans une direction radiale du corps de cylindre (31) pour diviser un espace (32) formé à l'intérieur du corps de cylindre (31) en une pluralité de chambres (33, 34) ; et
un élément de contrôle de l'écoulement (40) pour contrôler le mouvement du fluide entre la pluralité de chambres (33, 34),
dans lequel le module de commande fait fonctionner sélectivement l'appareil de prothèse de genou de type hybride (10) dans l'un quelconque des modes passif et actif en contrôlant l'entraînement de l'élément de contrôle de l'écoulement (40).

4. L'appareil de prothèse de genou de type hybride (10) de la revendication 3, dans lequel la lame est formée pour être en contact étroit avec la surface périphérique intérieure et la surface intérieure du corps de cylindre (31) de sorte que le mouvement du fluide entre la pluralité de chambres (33, 34) se fait uniquement par l'intermédiaire de l'élément de contrôle de l'écoulement (40).

5. L'appareil de prothèse de genou de type hybride (10) de la revendication 4, dans lequel, dans la lame, la surface de contact avec la surface périphérique intérieure et la surface intérieure du corps de cylindre (31) est formée d'un matériau en caoutchouc.

6. L'appareil de prothèse de genou de type hybride (10) de la revendication 3, dans lequel une première poulie à laquelle le câble d'entraînement est fixé est formée le long de la surface périphérique extérieure du corps de cylindre (31).

7. L'appareil de prothèse de genou de type hybride (10) de la revendication 3, dans lequel l'élément de contrôle de l'écoulement (40) comprend :
un boîtier disposé dans un espace formé à l'intérieur du corps de cylindre (31) et doté d'une voie d'écoulement à travers laquelle le fluide peut se déplacer ; et
une vanne de contrôle du débit formée à l'intérieur du boîtier, capable de contrôler le degré de mouvement du fluide se déplaçant dans une direction d'une chambre à l'autre parmi la pluralité de chambres (33, 34).

8. L'appareil de prothèse de genou de type hybride (10) de la revendication 7, dans lequel la vanne de régulation du débit comprend :
une première ouverture formée dans une partie de l'espace d'insertion de la buse à l'intérieur du boîtier, à travers laquelle le fluide peut se déplacer ; et
une buse intérieure insérée dans l'espace d'insertion de la buse et présentant une seconde ouverture correspondant à la première ouverture formée sur une surface périphérique extérieure de manière à ajuster le degré d'ouverture et de fermeture de la première ouverture en fonction de la rotation.

9. L'appareil de prothèse de genou de type hybride (10) de la revendication 8, dans lequel la valve de contrôle du débit comprend en outre un moteur de valve connecté à la buse intérieure pour faire tourner la buse intérieure.

10. L'appareil de prothèse de genou de type hybride (10) de la revendication 9, dans lequel la buse intérieure et le moteur de la valve sont reliés par un engrenage conique.

11. L'appareil de prothèse de genou de type hybride (10) de la revendication 7, dans lequel la vanne de régulation du débit comprend :
une première vanne de régulation du débit pour contrôler le degré de mouvement du fluide se déplaçant dans le sens d'une première chambre vers une deuxième chambre parmi la pluralité de chambres (33, 34) ; et
une deuxième soupape de régulation du débit pour contrôler le degré de mouvement du fluide se déplaçant dans la direction de la deuxième chambre vers la première chambre, afin de faire pivoter l'arbre de rotation (37) dans les deux directions de sorte que le module d'articulation du genou (20) puisse effectuer à la fois des mouvements de flexion et d'extension.

12. L'appareil de prothèse de genou de type hybride (10) de la revendication 7, dans lequel lorsque le mouvement du fluide entre la pluralité de chambres (33, 34) est bloqué par la vanne de contrôle du débit, la rotation pivotante de la lame est limitée, et lorsque l'ensemble du corps de cylindre (31) est mis en rotation par le câble d'entraînement, l'arbre de rotation (37) est mis en rotation pivotante en même temps pour faire fonctionner le mode actif.

13. L'appareil de prothèse de genou de type hybride (10) de la revendication 7, dans lequel lorsque le mouvement du fluide entre la pluralité de chambres (33, 34) est autorisé par la vanne de régulation du débit, lorsque la lame est tournée de manière pivotante par la pression hydraulique, l'arbre de rotation (37) est tourné de manière pivotante en même temps pour faire fonctionner le mode passif.

14. L'appareil de prothèse de genou de type hybride (10) de la revendication 13, dans lequel le module de commande contrôle la vitesse d'amortissement du module d'articulation du genou (20) en contrôlant le degré d'ouverture de la vanne de contrôle du débit en déterminant un environnement de marche en temps réel, sur la base d'informations de marche obtenues par un capteur monté sur l'appareil de prothèse de genou de type hybride (10).

15. L'appareil de prothèse de genou de type hybride (10) de la revendication 7, dans lequel l'élément de contrôle de l'écoulement (40) est pourvu d'un clapet anti-retour pour limiter la direction du mouvement du fluide à une seule direction à l'intérieur du boîtier.

16. L'appareil de prothèse de genou de type hybride (10) de la revendication 1, dans lequel un rotor est disposé à l'extérieur du moteur électrique (50), et une deuxième poulie à laquelle le câble d'entraînement est fixé est formée le long d'une surface périphérique extérieure du rotor.

17. L'appareil de prothèse de genou de type hybride (10) de la revendication 1, dans lequel un rotor est disposé à l'intérieur du moteur électrique (50), et une deuxième poulie à laquelle le câble d'entraînement est fixé est formée le long d'une surface périphérique extérieure d'un arbre d'entraînement relié au rotor.

18. L'appareil de prothèse de genou de type hybride (10) de la revendication 1, dans lequel, lorsque l'appareil de prothèse de genou est commandé en mode passif, l'entraînement du moteur électrique (50) est arrêté par un frein installé à l'intérieur.
